# EUROPÄISCHE PATENTANMELDUNG

(11) **EP 2 572 653 A1**
(43) Veröffentlichungstag der Anmeldung: **27.03.2013**
(21) Anmeldenummer: 12183311.5
(22) Anmeldetag: 06.09.2012
(51) Int. Cl.: A61B 17/064, A61B 17/00, A61B 19/02

(54) **Wundklammer**

(30) Priorität: 20.09.2011 DE 102011053781
(71) Anmelder: implantcast GmbH, 21614 Buxtehude (DE)
(72) Erfinder: Gebert, Eric, 48149 Münster (DE)
(74) Vertreter: Raffay & Fleck

(57) **Zusammenfassung**

Für eine Wundklammer (1, 1ʹ) zum Verschließen von Wunden bei tierischen oder menschlichen Patienten mit einem aus einem drahtartigen Material gebildeten, einen im wesentlichen geraden mittleren Abschnitt (2) und zwei sich an diesen anschließende gegenüber dem mittleren Abschnitt (2) abgewinkelt verlaufende im wesentlichen geraden Seitenabschnitte (3) aufweisenden Klammerkorpus, wird zur Verbesserung der Wundheilung einer mit einer solchen Wundklammer (1, 1ʹ) versorgten Wunde vorgeschlagen, dass die Oberfläche des Klammerkorpus in zumindest einem Bereich Kupfer enthält.

## Beschreibung

Die Erfindung betrifft eine Wundklammer zum Verschließen von Wunden bei tierischen oder menschlichen Patienten gemäß dem Oberbegriff des Patentanspruchs 1.

Wundklammern sind seit Jahrzehnten in der Medizin als ein mögliches Instrument zum Verschließen von Wunden bekannt. Sie werden als mögliche Alternative zu chirurgischem Nahtmaterial zum Verschließen von Wundöffnungen, sei es solchen, die beispielsweise durch Unfall erlitten sind, sei es solchen, die in Folge eines chirurgischen Eingriffes entstanden sind, verwendet.

Entsprechende Wundklammern werden, sofern sie Wundverschlüsse in oberen Gewebeschichten (insbesondere im Bereich der Haut und des unmittelbar darunter befindlichen Gewebes) betreffen, zum temporären Verbleib gesetzt und nach Verheilen der Wunde und Verwachsen der Wundränder entfernt. In tieferen Gewebeschichten, z.B. bei intraabdominellen Eingriffen werden entsprechende Wundklammern auch zum permanenten Verbleib gesetzt.

Für das Setzen der Wundklammern sind spezielle Geräte entwickelt, wie sie z.B. in der US 4,470,532 beschrieben sind. Vielfach handelt es sich hierbei um zur einmaligen Verwendung vorgesehene Geräte, die bereits mit Wundklammern in einer entsprechenden Anzahl befüllt und insgesamt steril verpackt ausgeliefert werden.

Die seit Jahrzehnten verwendeten Wundklammern sind in aller Regel aus medizinischem Stahl oder Titan gefertigt. Eine einzelne Wundklammer besteht somit aus einem Stahl- oder Titandraht, mit einem mittleren Abschnitt und zwei Seitenabschnitten, die winkelig zu dem mittleren Abschnitt verlaufen, insbesondere in einem Winkel von ca. 90° umgebogen sind. Dabei sind in der Regel die freien Enden der Seitenabschnitte angespitzt, um das Durchdringen von Gewebeschichten im Bereich der Wundränder der zu verschließenden Wunde zu erleichtern.

Es sind aber auch Wundklammern aus einem bioabsorbierbaren Kunststoff bekannt, die innerhalb des Körpers nach einer geraumen Verweildauer aufgelöst und absorbiert werden. Derartige Wundklammern sind z.B. in der EP 0 580 994 A1 beschrieben. Sie dienen einer Spezialanwendung, insbesondere zum Verschluss von Wundabschnitten in tiefer gelegenen Gewebebereichen, z.B. bei intraabdominellen Eingriffen. Dort werden die Wundklammern belassen, die Bioabsorbierbarkeit besorgt, dass diese nicht dauerhaft anzutreffen sind, sondern nach einer für das sichere Verheilen und Zusammenwachsen der Wundränder ausreichenden Zeitspanne aufgelöst werden.

Insbesondere bei den metallischen Wundklammern, aber auch bei solchen aus anderem Material hat sich herausgestellt, dass diese gegenüber einem Wundverschluss mit herkömmlichem Nahtmaterial gewisse Nachteile aufweisen. So besteht bei der Verwendung von Wundklammern aus medizinischem Stahl, welcher allgemein nickelhaltig ist bzw. sein kann, stets die Gefahr einer allergischen Reaktion des Patienten auf dieses als Allergen wirkende Metall. Darüber hinaus haben sich auch bei Verwendung anderer Materialien wiederholt Wundheilungsstörungen gezeigt, die auf die Tatsache, dass es sich bei den Wundklammern um größere Fremdkörper handelt, zurückgeführt werden. Insoweit ist - insbesondere klein dimensioniertes "mikrochirurgisches" Nahtmaterial der herkömmlichen Art den Wundklammern häufig überlegen, so dass trotz dem bestehenden Geschwindigkeitsvorteil bei der Versorgung - Wundklammern können besonders schnell und einfach gesetzt werden - vielfach der behandelnde Arzt auf das Setzen einer klassischen Naht mit chirurgischem Nahtmaterial zurückgreift und diese Vorgehensweise dem Einsatz von Wundklammern vorzieht.

In diesem Zusammenhang ist es Aufgabe der vorliegenden Erfindung, hier verbesserte Wundklammern anzugeben, die weniger Wundheilungsstörungen der so versorgten Wunden zeigen und vielmehr einen förderlichen Einfluss auf die Wundheilung nehmen.

Diese Aufgabe wird gelöst durch eine Wundklammer mit den Merkmalen des Patentanspruches 1. In den abhängigen Ansprüchen 2 bis 8 sind vorteilhafte Weiterbildungen einer erfindungsgemäßen Wundklammer angegeben. Ein weiterer Aspekt der Lösung der Aufgabe besteht in einer aus einer erfindungsgemäßen und sterilen Wundklammer in einer sterilen Umverpackung bestehenden Einheit. Schließlich wird als weiterer Aspekt der Lösung der Aufgabe ein Verfahren zum Herstellen einer Wundklammer mit den entsprechenden Eigenschaften angegeben.

Der wesentliche Ansatz der Erfindung besteht darin, zumindest in einem Abschnitt der Oberfläche des Korpus der Wundklammer ein kupferhaltiges Material aufzubringen bzw. diese Oberfläche aus einem solchen Material zu gestalten. Ein kupferhaltiges Material ist dabei insbesondere ein solches, welches in der Lage ist, bei zum Wundverschluss eingebrachter Wundklammer Kupferionen freizusetzen. Es hat sich nämlich herausgestellt, dass Kupferionen trotz der leicht toxischen Wirkung dieses Materials vom menschlichen bzw. tierischen Organismus gut vertragen wird, insoweit medizinisch unbedenklich ist. Darüber hinaus hat sich gezeigt, dass Kupfer (Kupferionen) einen proangiogenetischen Effekt erzielen, dadurch die Wundheilung und den Verschluss einer Wundöffnung bzw. das Zusammenwachsen von Wundrändern und die Ausbildung einer diese überbrückenden Gefäßstruktur zur Blutversorgung deutlich positiv beeinflussen. Auf diese Weise kann also die erfindungsgemäße Wundklammer den Wundheilungsprozess positiv beeinflussen, wodurch ein möglicher störender und den Heilungsprozess beeinträchtigender Effekt aufgrund des Vorhandenseins eines anderen Materials, wie er bei bekannten Wundklammern häufig zu erkennen war und dominierte, jedenfalls kompensiert wird, häufig sogar durch den proangiogenetischen Effekt des Kupfermaterials ins Gegenteil verkehrt wird. Der Einsatz erfindungsgemäßer Wundklammern führt mithin zu einer deutlich verbesserten Wundheilung so versorgter Wunden, so dass medizinische Komplikationen bedeutend seltener auftreten und die Heildauer entsprechend versorgter Wunden verkürzt werden kann, die erfindungsgemäßen Wundklammern mit geringerer Verweildauer verbleiben, also früher wieder entfernt werden können (jedenfalls dort, wo eine Entfernung vorgesehen ist). Entsprechend der Erfindung gebildete Wundklammern können aber auch zum dauerhaften Verbleib im Körper des Patienten vorgesehen sein, da auch ein Langzeiteinfluss des Kupfers der Oberfläche keine medizinisch bedenklichen Wirkungen auf den Organismus zeigt.

Sinnvollerweise sind bei der erfindungsgemäßen Wundklammer jedenfalls diejenigen Bereiche, die mit dem die Wunde umgebenden Gewebe in Kontakt gelangen, mit einer entsprechenden kupferhaltigen Oberfläche versehen. Bei einer oberflächlichen Wundversorgung (also im Bereich der Haut und der unmittelbar darunter liegenden Gewebeschichten) sind dies vorrangig die Seitenabschnitte, wobei hier aber auch der mittlere Abschnitt ebenfalls mit einer entsprechenden kupferhaltigen Oberfläche versehen sein kann. Bei solchen Wundklammern, die für den Einsatz im Körperinneren (z.B. zum Verschluss einer innerhalb des Abdomens liegenden Wunde nach einem intraabdominellen Eingriff) bestimmt sind, ist, da dort die gesamte Oberfläche der Wundklammer mit umgebenden Gewebe in Kontakt steht, idealerweise deren gesamte Oberfläche entsprechend kupferhaltig ausgebildet.

Die Ausbildung der Oberfläche mit Kupfer kann so sein, dass dort eine medizinisch unbedenkliche, Kupferionen freisetzende Kupferverbindung gewählt ist. Insbesondere enthält die Oberfläche des Klammerkorpus aber elementares Kupfer, was entweder eingebettet sein kann in einer Kunststoff- oder sonstigen Matrix, als reine Kupferbeschichtung oder reine Kupferoberfläche vorliegt oder aber in einer kupferhaltigen Legierung aufgebracht ist.

Der Klammerkorpus kann insbesondere aus einem Metalldraht gebildet sein. Dieser Metalldraht kann insgesamt ein aus reinem Kupfer oder einer kupferhaltigen Legierung gebildeter Metalldraht sein, mit einer im wesentlichen homogenen Struktur, mit anderen Worten mit einer gleichen Materialzusammensetzung im Inneren wie an seiner Oberfläche. Es kann aber auch als Metalldraht ein erstes metallisches Trägermaterial gewählt sein, welches insbesondere nicht kupferhaltig zu sein braucht, welches dann mit einer kupferhaltigen Oberfläche versehen bzw. beschichtet ist, in zumindest dem einen Bereich. Mögliche metallische Trägerstrukturen sind dabei CoCrMo-Gusslegierungen nach ASTM F 75 bzw. ISO 5832-4, CoCrMo-Schmiedelegierungen nach ISO 5832-12, CoNiCrMo-Schmiedelegierungen nach ASTM F 536 bzw. ISO 5832-6 sowie Titan-Legierungen nach ISO 5832-3 und 5832-11. Diese entsprechenden Legierungen können auch Legierungspartner darstellen, denen zur Herstellung einer kupferhaltigen Oberfläche Kupfer beigemengt wird.

Grundsätzlich ist es aber auch möglich, den Klammerkorpus insgesamt aus elementarem Kupfer oder einer Kupferlegierung zu bilden. Da elementares Kupfer jedoch von vergleichsweise geringer Festigkeit ist, bietet sich insbesondere dann, wenn die Wundklammer hohe Haltekräfte aufzubringen hat, um die Wundränder geschlossen zu halten, der Einsatz einer Kupferlegierung mit höherer Festigkeit an.

Mit Vorteil ist die Oberfläche des Klammerkorpus vollständig nickelfrei gebildet. Eine solche Vorgehensweise verhindert, dass bei nickelempfindlichen Patienten allergische Reaktionen auf das Vorhandensein dieses Materials in Kontakt mit dem umliegenden Gewebe ausgelöst werden und damit einhergehende Komplikationen auftreten.

Selbstverständlich sind die erfindungsgemäßen Wundklammern steril. Nur so können sie im medizinischen bzw. chirurgischen Umfeld eingesetzt werden.

Um die Sterilität der Wundklammer(n) zu erhalten wird mit der Erfindung auch eine Einheit aus einer oder mehrerer steriler Wundklammer(n) in einer diese umschließenden sterilen Umverpackung vorgeschlagen. Dabei ist unter einer solchen Umverpackung einerseits eine unmittelbare, die Wundklammer bzw. Wundklammern umgebende Verpackung zu verstehen, aus der diese Wundklammer bzw. Wundklammern entnommen und dann in ein medizinisches Wundklammergerät überführt werden. Alternativ kann eine solche sterile Umverpackung auch ein (Einweg-) Wundklammergerät mit umfassen, in welchem die erfindungsgemäßen Wundklammern angeordnet sind. Das Wundklammergerät ist in diesem Zusammenhang dann Bestandteil der sterilen Umverpackung.

Ein weiterer Aspekt der Erfindung besteht in einem Verfahren zum Herstellen einer Wundklammer aus einem Abschnitt eines drahtartigen, zumindest in einem Bereich seiner Oberfläche Kupfer, insbesondere elementares Kupfer, aufweisenden Materials durch Umbiegen seiner Enden, wobei die Wundklammer unter sterilen Bedingungen aus dem zuvor bereits sterilisierten Materialabschnitt hergestellt wird und/oder zum Abschluss des Herstellungsprozesses sterilisiert wird.

Der wesentliche Aspekt der Erfindung besteht, um dies noch einmal zusammenzufassen, darin, dass Kupfer als Element gezielt an der Oberfläche der neuartigen Wundklammer eingesetzt wird, um die besonderen Effekte dieses Materials im Zusammenhang mit der Wundheilung zu nutzen. Diese Effekte sind neben der bereits beschriebenen proangiogenetischen Wirkung, die die Wundheilung generell verbessert, auch eine mögliche Unterdrückung von Allergien (bei nickelfrei ausgebildeter Oberfläche), da Kupfer hypoallergen ist, ferner eine antimikrobielle Wirkung der Kupferionen, womit im Bereich der Wundränder und der Wunde möglicherweise befindliche Keime abgetötet werden und eventuellen Entzündungen vorgebeugt wird, sowie ein geringer Ferromagnetismus des Materials Kupfer (wenn dies in besonders hohem Anteil Verwendung findet) womit geringe Artefakte im MRT bei in situ Anwendungen erreicht werden. Grundsätzlich ist es auch möglich, eine kupferhaltige Oberfläche bei bekannten Kunststoffwundklammern zu verwenden, z.B. solchen die bioabsorbierbar sind, wie etwa die in der oben bereits erwähnten EP 0 580 994 A1 offenbarten. Auch hier kann das Einbringen von Kupfer an der Oberfläche der entsprechenden Wundklammer mit seiner proangiogenetischen und antimikrobiellen Wirkung den Wundheilungsprozess befördern und somit medizinisch positiv wirken.

Weitere Vorteile und Merkmale der Erfindung ergeben sich aus der nachfolgenden Beschreibung eines möglichen Ausführungsbeispiels anhand der beigefügten Figuren. Dabei zeigen:
- Fig. 1: in einer schematischen Ansicht übereinander angeordnet zwei Beispiele von Wundklammern, deren Oberfläche in erfindungsgemäßer Weise ebenfalls in einem Bereich mit kupferhaltigem Material gebildet sind, in einer Ausgangsstellung;
- Fig. 2: die beiden in Fig. 1 gezeigten Wundklammern in einer Stellung, wie sie nach dem Applizieren geformt sind;
- Fig. 3: die Position der Wundklammern im Gewebe, hier am Beispiel der Anbringung in zwei übereinander angeordneten Gewebeschichten; und
- Fig. 4: in einer schematischen Darstellung eine nach Applikation und Umformen in anderer Form im Gewebe angeordnete Wundklammer.

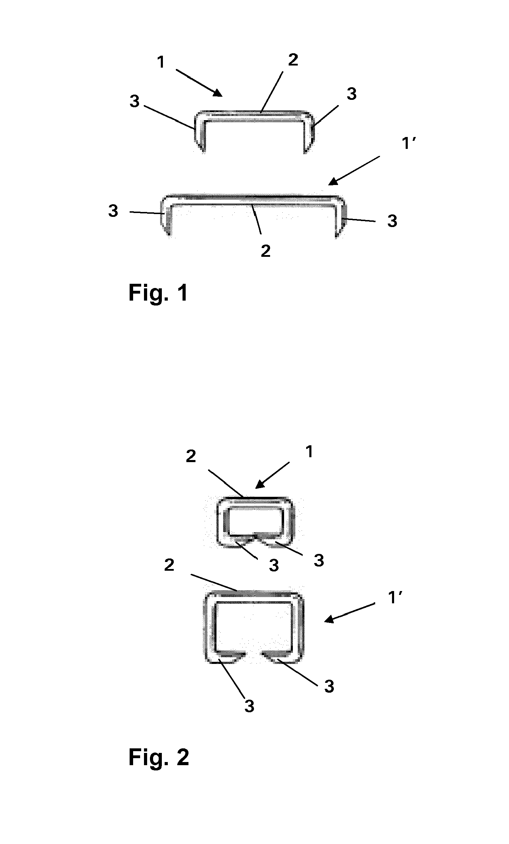

In Fig. 1 sind zwei unterschiedlich bemaßte Beispiele für erfindungsgemäße Wundklammern gezeigt, eine kleiner dimensionierte Wundklammer 1 sowie eine größer dimensionierte Wundklammer 1'. Beide Wundklammern sind mit ihrem Klammerkorpus aus einem drahtartigen Material geformt und weisen einen langgestreckten mittleren Abschnitt 2 sowie zwei sich daran anschließende Seitenabschnitte 3 auf, die jeweils wieder im Wesentlichen geradlinig verlaufen, gegenüber dem mittleren Abschnitt 2 aber winkelig gelegen sind, insbesondere in einem Winkel von etwa 90°. An den freien Enden der Seitenabschnitte 3 sind diese spitz zulaufend angeschrägt, um ein Eindringen der Wundklammern mit diesen Abschnitten 3 in das die Wunde umgebende Gewebe zu erleichtern. Die in Fig. 1 gezeigten Wundklammern sind in erfindungsgemäßer Weise zumindest in einem Bereich der Abschnitte 2 und/oder 3 mit einer kupferhaltigen Oberfläche versehen, sie können dort insbesondere elementares Kupfer enthalten, sei es in Form einer reinen Kupferoberfläche oder aber einer Oberfläche einer kupferhaltigen Legierung. Die Kupferhaltigkeit der Oberfläche zeichnet sich allgemein dadurch aus, dass die Oberfläche in der Lage ist, Kupferionen im Gewebe freizusetzen und an die Umgebung abzugeben. Insbesondere ist die gesamte Oberfläche der Wundklammern 1 bzw. 1' mit einem kupferhaltigen Überzug versehen bzw. enthält Kupfer in einer zur Abgabe von Kupferionen geeigneten Form.

Der drahtartige Korpus der Wundklammern 1 bzw. 1' kann dabei insgesamt metallisch sein, die Wundklammern 1 bzw. 1' können durchgehend aus einem homogenen Material, beispielsweise elementarem Kupfer oder einer Kupferlegierung, bestehen. Auch ist es möglich, dass der metallische Korpus einen Kern aus einem ersten metallischen Material, z.B. einer CoCrMo-Legierung aufweist, der mit einer kupferhaltigen Oberfläche beschichtet ist. Ebenso gut kann der Korpus der Wundklammern 1 bzw. 1' ein Kunststoffkorpus sein, auch aus einem bioabsorbierbarem Kunststoff, wobei auf der Oberfläche Kupfer eingebracht ist, z.B. durch Einbetten in eine Kunststoffmatrix, welches Kupfer dann im Applikationsbereich der Wundklammer freigesetzt werden kann in Form von Kupferionen.

In Fig. 2 sind die in Fig. 1 in einer Ausgangsstellung gezeigten Wundklammern 1 bzw. 1' in einer Form gezeigt, wie sie nach der Applikation im Gewebe zum Verschließen eines Wundrandes eingestellt ist. Zu erkennen ist, dass die Wundklammern jeweils im Bereich ihres mittleren Abschnittes 2 durch eine Applikationsvorrichtung eine weitere Umbiegung in einem Winkel von etwa 90° erfahren, so dass die Wundklammer ein O- bzw. C-förmiges, im Wesentlichen geschlossenes Profil erhält, in dem sie mit ihren seitlichen Abschnitten 3 den Wundbereich zum Schluss der Wunde untergreifen und zusammenhalten können.

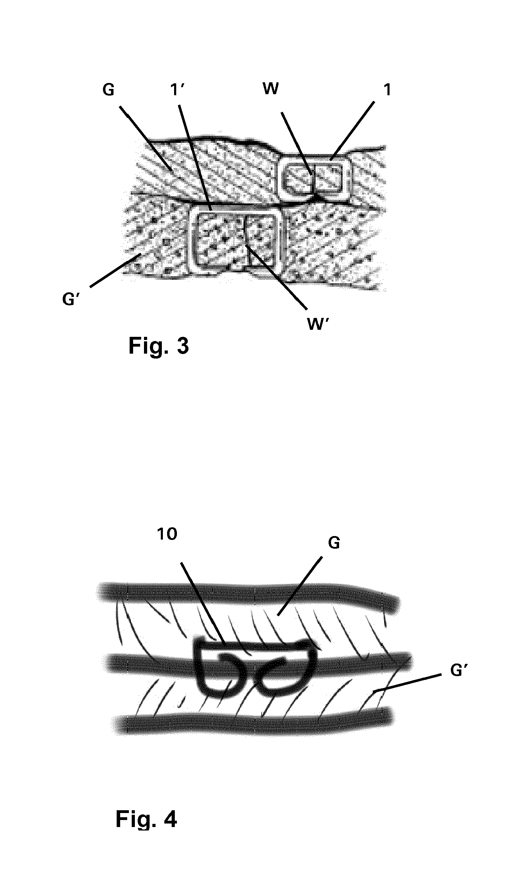

In Fig. 3 ist ein Anwendungsbeispiel für erfindungsgemäße Wundklammern gezeigt, die Wundklammer 1 ist hier zum Verschließen einer ersten Wunde W im Bereich einer oberen Gewebeschicht G eingesetzt, die Wundklammer 1' sitzt im Bereich einer zweiten Wunde W' in einer tiefer gelegenen Gewebeschicht G'. Während die Wundklammer 1 nach Ausheilen der Wunde W typischerweise entfernt wird, ist die Wundklammer 1' zum Verbleib vorgesehen, auch wenn die Wunde W' verheilt ist. Sie ist ohne ein erneutes Öffnen der Wunde W nicht mehr zugänglich, so dass eine Entfernung dieser Wundklammer typischerweise unterbleibt. Entsprechend kann die Wundklammer 1' aus einem bioabsorbieren Kunststoff bestehen, dessen Oberfläche mit Kupfer bzw. kupferhaltigem Material versehen und entsprechend ausgebildet ist. Die Wundklammer 1 wird in dieser Anwendung typischerweise eine Metallklammer sein, die ebenfalls eine kupferhaltige Oberfläche aufweist. Während der Verweildauer der Wundklammern 1 bzw. 1' im Bereich der Wunden W bzw. W' wird aufgrund der kupferhaltigen Oberfläche von dort permanent ionenförmiges Kupfer freigesetzt, welches aufgrund seiner proangiogenetischen Wirkung die Ausbildung einer Gefäßversorgung im Bereich der Wunde und diese übergreifend fördert und damit insgesamt die Wundheilung verbessert und beschleunigt. Durch seine zusätzliche antimikrobielle Wirkung hilft das freigesetzte Kupfer, im Bereich der versorgten Wunden W bzw. W' Keime abzutöten und damit eventuelle Entzündungen zu verhindern.

Die gezeigten Ausführungsbeispiele sind rein beschreibend und beschränken den Schutzumfang der Erfindung, wie er in den nachfolgenden Ansprüchen bestimmt wird, nicht. Insbesondere sind Wundklammern anderer Formen und Dimensionsverhältnisse ebenfalls mit von der Erfindung umschlossen, wie auch solche Wundklammern, die z.B. im Verlauf ihrer mittleren Abschnitte 2 nicht exakt gradlinig verlaufen, sondern Krümmungen erfahren.

In Fig. 4 schließlich ist schematisch eine weitere Wundklammer 10 nach Applikation im Gewebe angeordnet dargestellt. Diese hat abweichend von dem in Fig. 3 gezeigten C- bzw. O-Profilen nach der während der Applikation erfolgten Umformung eine B-Form. Diese Form, die durch das Zusammenwirken der Wundklammer mit dem entsprechenden Applikationsgerät erzielt wird, ist insbesondere für die Anwendung von Wundklammern im intraabdominellen Bereich heute weit verbreitet.

### Bezugszeichenliste

- 1: Wundklammer
- 1': Wundklammer
- 2: mittlerer Abschnitt
- 3: Seitenabschnitt
- 10: Wundklammer

- G: Gewebeschicht
- G': Gewebeschicht
- W: Wunde
- W': Wunde

## Patentansprüche

1. Wundklammer zum Verschließen von Wunden bei tierischen oder menschlichen Patienten mit einem aus einem drahtartigen Material gebildeten, einen im wesentlichen geraden mittleren Abschnitt (2) und zwei sich an diesen anschließende gegenüber dem mittleren Abschnitt (2) abgewinkelt verlaufende Seitenabschnitte (3) aufweisenden Klammerkorpus, **dadurch gekennzeichnet, dass** die Oberfläche des Klammerkorpus in zumindest einem Bereich Kupfer enthält und dass die Oberfläche des Klammerkorpus vollständig nickelfrei gebildet ist und in der Lage ist, bei zum Wundverschluss eingebrachter Wundklammer Kupferionen freizusetzen.

2. Wundklammer nach Anspruch 1, **dadurch gekennzeichnet, dass** der Klammerkorpus in dem zumindest einen Bereich seiner Oberfläche elementares Kupfer enthält.

3. Wundklammer nach einem der vorhergehenden Ansprüche, **dadurch gekennzeichnet, dass** die Oberfläche des Klammerkorpus vollständig aus einem kupferhaltigen Material oder einer solchen Materialzusammensetzung gebildet ist.

4. Wundklammer nach einem der vorhergehenden Ansprüche, **dadurch gekennzeichnet, dass** der Klammerkorpus aus einem Metalldraht gebildet ist.

5. Wundklammer nach einem der vorhergehenden Ansprüche, **dadurch gekennzeichnet, dass** der Klammerkorpus einen Kunststoffdraht umfasst, der an seiner Oberfläche in zumindest dem einen Bereich Kupfer enthält.

6. Wundklammer nach Anspruch 4, **dadurch gekennzeichnet, dass** der Klammerkorpus einen Kern aus einem ersten nicht kupferhaltigen Material aufweist und in zumindest dem einen Bereich eine metallische Beschichtung aus elementarem Kupfer oder einer Kupferlegierung enthält.

7. Wundklammer nach Anspruch 4, **dadurch gekennzeichnet, dass** der Klammerkorpus einheitlich aus elementarem Kupfer oder einer Kupferlegierung besteht.

8. Wundklammer nach einem der vorstehenden Ansprüche, **dadurch gekennzeichnet, dass** die Seitenabschnitte gerade verlaufen.

9. Einheit bestehend aus einer oder mehrerer steriler Wundklammer(n) (1, 1') nach einem der vorhergehenden Ansprüche in einer diese umschließenden sterilen Umverpackung.

10. Verfahren zum Herstellen einer Wundklammer aus einem Abschnitt eines drahtartigen, zumindest in einem Bereich seiner Oberfläche Kupfer, aufweisenden Materials, wobei die Oberfläche des Klammerkorpus vollständig nickelfrei gebildet ist und in der Lage ist, bei zum Wundverschluss eingebrachter Wundklammer Kupferionen freizusetzen, durch Umbiegen seiner Enden, wobei die Wundklammer unter sterilen Bedingungen aus dem zuvor bereits sterilisierten Materialabschnitt hergestellt wird und/oder zum Abschluss des Herstellungsprozesses sterilisiert wird.
